# EUROPEAN PATENT APPLICATION

(11) **EP 3 417 843 A1**
(43) Date of publication of application: **26.12.2018**
(21) Application number: 17892058.3
(22) Date of filing: 28.03.2017
(51) Int. Cl.: A61J 3/07, A23L 2/52, A61K 47/00

(54) **PACK OF FLAVOUR ADDITIVES FOR ORAL ENTERAL NUTRITION**

(71) Applicant: ADVENTIA PHARMA, S.L., 35002 Las Palmas de G.C. (ES)
(72) Inventor: DIAZ ORTEGA, Jose Javier, 35002 Las Palmas de Gran Canaria (ES)
(74) Representative: Del Valle Valiente, Sonia
(86) International application number: PCT/ES2017/070180
(87) International publication number: WO 2018/178406

(57) **Abstract**

Flavoring additive kit for oral enteral nutrition for incorporating, upon consumption, into a basic product of an oral enteral diet and modifying its scent and flavor, consisting of a pack (1) comprising a set of between two and up to ten capsules (2) containing single doses of flavoring additives (3) each one of different flavors; flavors such as tofu, forest fruits, rice pudding, banana, cookie and lemon. The capsules (2) preferably contain single doses of liquid flavoring additives (3) and the pack (1) is a blister pack of plastic material with the capsules (2) defined by beveled cavities of the same material located in separable portions with an opening system by means of tearing off one weakened point (4) and a pouring system of the content by dropping when the same are repeatedly pressed.

## Description

### OBJECT OF THE INVENTION

The invention, as expressed in the title of the present specification, relates to a flavoring additive kit for oral enteral nutrition which provides advantages and novel characteristics for its intended function which will be described in detail below and which involve an improvement to the prior art.

In particular, the object of the invention is centered on a pack comprising a set of capsules which contain single doses of different flavoring additives to incorporate, upon consumption, into a basic product of an oral enteral diet which are marketed together in said pack forming a kit of various single doses of different flavors which allow the user to choose each time the one they prefer in order to change the flavor of the diet as desired and which also allow the intensity of the flavor to be change in a controlled manner and as desired.

### FIELD OF APPLICATION OF THE INVENTION

The field of application of the present invention is within the sector of the industry dedicated to the manufacture of medical supplies, with a particular focus on the range of products for an oral enteral diet.

### BACKGROUND OF THE INVENTION

As is known, enteral nutrition is a special feeding technique, which together with parenteral nutrition, is called artificial nutrition and consists of administering the different nutritional elements by way of a tube which is placed such that one end remains on the exterior and the other on some point of the digestive tract such as the stomach, duodenum or jejunum, eliminating the oral, esophageal and digestive steps. However, within a broader concept, enteral nutrition also includes the oral route if nutritional supplements or chemically-defined formulas are used, this enteral nutrition being the type affected by the object of the present invention.

One of the main drawbacks of these types of diets is that they are a product with limited variety of flavors and texture. This means that their intake is monotonous and less appealing, reducing the possibility of success of the treatment. The patient has few options to choose from and cannot interact with the product. In order to resolve this, flavoring additives can be incorporated into the basic product which provide different scents and flavors such that the range of options available for selection by the user is widened. This does not involve any drawback for the treatment and impacts beneficially to a certain degree on said treatment since it at least improves the mood of the patient when they eat it and increases the percentage of compliance with the treatments.

The object of the present invention is thus to develop a means which allows said problem to be solved for these types of diets, meaning that the patients can change the flavor of the additive and the intensity which they freely incorporate into their diet any time and thereby can change the flavor of the same as many times as they want in order to avoid it being monotonous and plain.

Moreover, another of the problems presented by these types of diets is that, depending on the pathology, the palatability of the patients may vary significantly, consequently, not all of them notice the flavor of the additive added to the diet with the same intensity, and therefore, they need more or less of the quantity of the additive in order to notice it. Therefore, it would also be desirable to be able to have a means so that the patient themselves can freely choose said intensity.

In addition and by way of reference to the prior art, it should be pointed out that the applicant, at least, does not know of the existence of another kit of flavoring additives for enteral nutrition nor another invention with similar application which has technical and constructive characteristics identical or similar to those which the outlined and claimed kit specifically has.

### DESCRIPTION OF THE INVENTION

The kit of flavoring additives for oral enteral nutrition, which the invention proposes, is configured, as a notable novelty within its field of application since, in terms of its implementation and in a limited manner, the objectives previously indicated as ideal are satisfactorily achieved, the characterizing details which make it possible and which conveniently distinguish it being captured in the final claims accompanying the present description.

Specifically, what the invention proposes, as has been noted above, is a pack comprising a set of capsules which contain single doses of different specific flavoring additives to incorporate, upon consumption, into a basic product of an oral enteral diet, which are marketed together in said pack forming a kit of various single doses of different flavors, such as at least two, advantageously allowing the user to choose the one they prefer each time they have to consume their diet and thereby allow the flavor to be freely changed so that it is not tasteless and monotonous.

In the preferred embodiment, the kit is a blister pack with five or six capsules filled with doses of liquid flavoring additives each one with different flavors, such as for example tofu, forest fruits, rice pudding, banana, cookie and lemon, but without being limited since other flavors and options of the flavoring additives in powder, granulated or another form are not ruled out.

In any case, in the preferred embodiment, the additives are liquid and the pack is a blister pack of plastic material where the capsules are defined by opening cavities by means of tearing and pouring drops when the capsules are pressed, therefore, the kit, in addition to allowing the selection of the flavor of the additive to be mixed, allows the quantity of the additive added to the basic product to be controlled. Specifically by pressing on said cavity to a greater or lesser extent, between one and three, to force the falling of all or only one part of its content. The capacity of each capsule is preferably approximately 1 ml and they are implemented in a material such as polyethylene with a twist-off opening system.

Therefore, the following improvements are achieved with respect to the forms of presentation and administration of diets for oral enteral nutrition available at present on the market:
It gives the user a much wider range of flavors based on the diet of basic flavors and allows the user to decide the flavor to be obtained each time. This possibility makes this diet more varied and attractive, contributing to greater efficiency of the treatment as a therapeutic instrument.

In patients with reduced palatability as a result of determined pathologies, the possibility of increasing the intensity of the flavor by means of providing an additive allows the effects mentioned in the previous point to be achieved.

The way of the patient proceeding to use the kit of the invention will be as follows:
- the oral enteral diet pack with the base product is opened.
- the capsule with the chosen flavoring additive is selected in the kit, for which each capsule is conveniently identified with a label or similar.
- the chosen capsule is separated from the rest of the pack.
- the capsule is opened and the content of the single dose is added in the base product of the oral enteral diet. The intensity of the new flavor can be modulated depending on the quantity of drops added, for which one, two or three presses of the capsule are carried out which determine the dropping of a greater or lesser part of the dose or the complete dose of the additive.
- the oral enteral nutrition pack is closed and is stirred to achieve the correct mixing of the additive.
- the oral enteral diet remains ready for its administration.

Optionally, more than one dose of flavoring additive can be added to the base product as well as mixing a dose or portions of a dose of two or more different flavoring additives which increases the possibilities of varying the flavor and intensity of the diet for the user during each consumption.

In light of the foregoing, it should be stated that the described kit of flavoring additives for oral enteral nutrition represents an innovation of structural and constructive characteristics unknown up to now for the intended purpose, which is why, together with its practical use, they give the invention sufficient grounds to obtain the privilege of exclusivity requested.

### DESCRIPTION OF THE DRAWINGS

In order to complement the description being made and with the object of helping to better understand the characteristics of the invention, in accordance with a preferred practical embodiment thereof, said description is accompanied, as an integral part thereof, by a sheet of figures where, in an illustrative and non-limiting manner, the following has been represented:

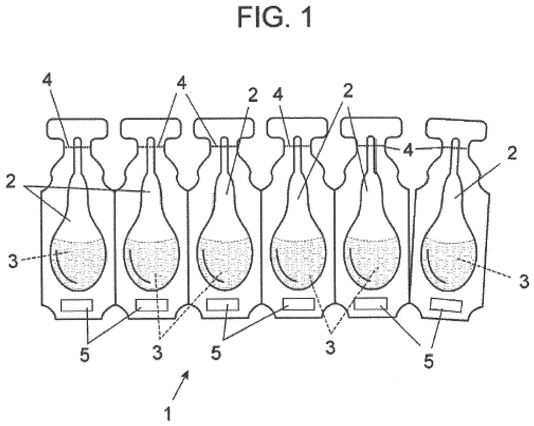

Figure 1 shows a schematic view of an elevation of an exemplary embodiment of the kit of flavoring additives for oral enteral nutrition, object of the invention, its general configuration and the main parts forming it being discernible.

### PREFERRED EMBODIMENT OF THE INVENTION

In view of the described Figure 1 and in accordance with the numeration adopted therein, a non-limiting example of the kit of flavoring additives for oral enteral nutrition of the invention can be discerned which comprises the parts and elements indicated and described in detail below.

As is observed in said Figure 1, the kit in question consists of a pack (1), preferably a blister type pack, comprising a set of at least two capsules (2) containing single doses of flavoring additives (3) each one with different flavors, specifically suitable for incorporating, upon consumption, into a basic product of an oral enteral diet, said product consisting of a nutritional formula prepared industrially which is used to compensate the deficiency in patients with poor nutrition, caused by a reduction in normal ingestion or by pathological processes and said flavoring additives consisting of a preparation of natural essences which change the scent and flavor of the oral enteral diet upon being mixed therewith.

In the preferred embodiment, the pack (1) constituting the kit of the invention comprises capsules (2) filled with single doses of liquid flavoring additives (3) each one with different flavors, preferably between five and six and up to a maximum of ten, consisting of flavors such as for example tofu, forest fruits, rice pudding, banana, cookie and lemon.

In any case, the pack (1) is preferably a blister type pack of plastic material such as polyethylene formed by thermo-molding where the capsules (2) are defined by beveled cavities of the same material of said pack (1) located singly in portions separable from the set, as is observed in the example represented in Figure 1 and each one of said capsules (2) has an opening system by means of tearing off one point (4) weakened for such purpose on the same and a pouring system of the content by dropping when the same are repeatedly pressed, the capacity of each capsule (2) preferably being approximately 1 ml.

Lastly, it should be mentioned that each capsule (2) also incorporates a label or mark, signal, color or identification (5) of the flavor of each one of the different flavoring additives (3) which each capsule (2) contains so that the user can distinguish them.

With the nature of the present invention sufficiently described and the manner of putting it into practice, it is not considered necessary to give a more extensive explanation for a person skilled in the art to understand its scope and the advantages derived from it, it being stated that it can be carried out in practice in other embodiments which differ in detail from what is indicated by way of example provided it is not fundamentally changed.

## Claims

1. A FLAVORING ADDITIVE KIT FOR ORAL ENTERAL NUTRITION, particularly flavoring additives for incorporating, upon consumption, into a basic product of the oral enteral diet, said product consisting of a nutritional formula prepared industrially which is used to compensate the deficiency in patient with poor nutrition, caused by a reduction in normal ingestion or by pathological processes and said flavoring additives consisting of a preparation of natural essences which change the scent and flavor of the oral enteral diet upon being mixed with said product, **characterized by** consisting of a pack (1) comprising a set of at least two capsules (2) containing single doses of flavoring additives (3) each one of different flavors.

2. THE FLAVORING ADDITIVE KIT FOR ORAL ENTERAL NUTRITION according to claim 1, **characterized in that** the pack (1) comprises five or six capsules (2) filled with single doses of flavoring additives (3) each one with different flavors.

3. THE FLAVORING ADDITIVE KIT FOR ORAL ENTERAL NUTRITION according to claim 2, **characterized in that** the different flavoring additives (3) which the capsules (2) contain are flavors such as tofu, forest fruits, rice pudding, banana, cookie and lemon.

4. THE FLAVORING ADDITIVE KIT FOR ORAL ENTERAL NUTRITION according to claim 1, **characterized in that** the pack (1) comprises up to 10 capsules (2) filled with single doses of flavoring additives (3) each one with different flavors.

5. THE FLAVORING ADDITIVE KIT FOR ORAL ENTERAL NUTRITION according to any of claims 1 to 4, **characterized in that** the capsules (2) are filled with single doses of liquid flavoring additives (3).

6. THE FLAVORING ADDITIVE KIT FOR ORAL ENTERAL NUTRITION according to claim 5, **characterized in that** the pack (1) is a blister type pack of plastic material such as polyethylene.

7. THE FLAVORING ADDITIVE KIT FOR ORAL ENTERAL NUTRITION according to claim 6, **characterized in that** the capsules (2) are defined by beveled cavities of the same material of said pack (1) located singly in portions separable from the set and have an opening system by means of tearing off one point (4) weakened for such purpose on the same and a pouring system of the content by dropping when the same are repeatedly pressed.

8. THE FLAVORING ADDITIVE KIT FOR ORAL ENTERAL NUTRITION according to claim 7, **characterized in that** the capacity of each capsule (2) is approximately 1 ml.

9. THE FLAVORING ADDITIVE KIT FOR ORAL ENTERAL NUTRITION according to any of claims 1 to 8, **characterized in that** each capsule (2) also incorporates a label or mark, signal, color or identification (5) of the flavor of each one of the different flavoring additives (3) which each capsule (2) contains so that the user can distinguish them.
